# EUROPEAN PATENT SPECIFICATION

(11) **EP 1 539 053 B1**
(45) Date of publication and mention of the grant of the patent: **13.02.2008**
(21) Application number: 03754568.8
(22) Date of filing: 12.09.2003
(51) Int. Cl.: A61F 2/46, A61F 2/44

(54) **IMPLANT MANIPULATION AND STORAGE TOOLS**
WERKZEUGE ZUR HANDHABUNG UND AUFNAHME EINES IMPLANTATS
OUTILS DE MANIPULATION ET DE STOCKAGE D'IMPLANTS

(30) Priority: 13.09.2002 US 410458 P; 05.11.2002 US 423864 P
(43) Date of publication of application: 15.06.2005
(60) Divisional application: 06013152.1
(73) Proprietor: Replication Medical, INC., New Brunswick, NJ 08901 (US)
(72) Inventor: STOY, Vladimir, 25267 Praha Zapad (CZ); DICKHUDT, Eugene, A., New Brighton, MN 55112 (US); ZIEBOL, Robert, Blaine, MN 55434 (US); LOVY, Jan, P.O. Box 253 Rocky Hill, NJ 08553 (US); GONTARZ, Gerald, Spotswood, NJ 08884 (US); BAKER, Dan, Suite 101 Seattle, WA 98125 (US)
(74) Representative: Lorenz, Eduard
(86) International application number: PCT/US2003/028878
(87) International publication number: WO 2004/024039

(56) References cited:
- EP-A- 1 114 623
- WO-A-02/060371
- FR-A- 2 808 993
- US-A- 5 304 182
- US-A- 5 993 459
- US-A1- 2001 002 440
- US-A1- 2001 010 021
- US-A1- 2001 032 020
- US-A1- 2002 116 006
- US-A1- 2002 123 808

## Description

### BACKGROUND

### 1. Technical Field

The present disclosure relates to tools for flexible implants, and more particularly, to manipulation and storage tools for use with flexible gel implants.

### 2. Background of Related Art

Certain spinal conditions can result in severe pain to a patient as a result of a protruding intervertebral disk or a degenerative disk positioned between adjacent vertebrae.

Various surgical procedures are known to attempt to correct the appropriate spacing between the adjacent vertebrae and minimize the impact of the damaged disk on the adjacent spinal cord. One such solution includes the affixation of one or more external rods to the adjacent vertebrae to fix the adjacent vertebrae in a proper spacing and retain them in that position. Alternatively, various internal devices have been designed which are positioned within an excised portion of the intervertebral disk and are configured to fuse or lock up the adjacent vertebrae in order to relieve any pressure on the spine. While both these procedures are quite common, they do have the disadvantage of fusing or locking the adjacent vertebrae resulting in reduced or limited flexibility in that area of the spine as well as taking an extended period of recovery.

Another known procedure for relieving pressure on the spine, due to a defective intervertebral disk, involves the placement of a partial or whole replacement implant or disk in the intervertebral disk space which allows flexibility of the spine to be maintained while maintaining the adjacent vertebrae in their proper spacing or disk height.

In these procedures, the disk implant is typically in a partially hydrated and flexible state and is manipulated by various hand instruments or tools to configure it into a folded or compressed shape state while it is being inserted into the disk space. Once the compressed implant is put into the disk space it is allowed to expand or regain its original shape and re-hydrate to an appropriate restored disk height.

Problems occur during manipulation of the disk into a compressed state and in trying to maintain that particular compressed state while the disk is being manually inserted into the intervertebral disk space. Proper and precise placement of the disk in a position to re-hydrate to a proper height is also one of the difficulties in performing the substitute disk procedure manually. Thus, it would be desirable to have a tool which could precisely manipulate the partially dehydrated implant into a specific compressed shape. It would further be desirable to take the compressed disk and load it into a storage member for relatively quick use and precise insertion. Additionally, it would be desirable to have an insertion tool configured to receive the stored and compressed disk, precisely position it adjacent the vertebrae and insert it between the vertebrae so that it can re-hydrate and expand to a proper disk height.

From US 2002/0123808 A1 an apparatus for folding a flexible implant is known, in which the implant is pulled from a carrier cartridge in which the flexible implant can be stored in flat position, through folding die into a deployment cannula by strings attached to the flexible implant. By pulling the implant through the folding die, the implant can be reduced in size. The implant can then be inserted between the vertebrae using the deployment cannula. However, this method necessitates special implants having preformed strings. Additionally, the manipulation of the apparatus is complicated and imprecise.

The aim of the present invention is therefore to provide surgical instrument assembly which is easier to use and which has better precision.

This object is accomplished by a surgical instrument assembly according to claim 1 including a surgical instrument and a transfer tube along with a working sleeve.

The transfer tube for receipt of a folded implant and for use with the surgical instrument assembly preferably includes a transfer sleeve for receipt of the folded implant and first and second nuts rotatably upon the sleeve. The first nut is configured to engage the distal end of a surgical instrument and the second nut is configured to cam a lock member against a working sleeve.

The surgical instrument configured to drive a folded implant into the body generally includes a body portion having a fixed handle and a movable handle. The surgical instrument includes a pusher rod movable through the body portion in response to actuation of the movable handle. Ratchet mechanisms are provided to bias the pusher rod in a distal direction and prevent inadvertent retraction of the rod.

There is disclosed a manipulation and storage tool not forming part of the scope of protection of this patent for receiving a partially dehydrated flexible implant, manipulating it to a smaller or compressed overall size and inserting it into a storage or sleeve device. The manipulation and storage tool generally includes a guide member or assembly and a manipulation assembly movably mounted with respect to the guide assembly. The guide assembly includes a chamber or longitudinal throughbore and an opening or slot for receipt of a disk so that the disk extends across the throughbore. Preferably, a storage or sleeve holder is mounted to the distal end of the guide assembly to frictionally engage and retain a storage member or sleeve therein.

The manipulation assembly generally includes a drive member having an elongated outer tube extending distally into the guide assembly. A pair of manipulation members or pins extend distally from the distal end of the outer tube and are positioned adjacent the slot to receive the disk therebetween. The drive member has a circumferential track including a level portion extending approximately 180° around the drive member and an angled portion extending from the distal end of the drive member towards a proximal end of the drive member the remaining 180° around the drive member. A longitudinal portion connects the level portion of the track with the proximal end of the angle portion of the track. The movement of the manipulation assembly relative to the guide assembly is controlled by a drive rod fixedly mounted to a proximal end of the guide assembly and having a drive pin which resides in the track of drive member. Thus, as the drive member is rotated an initial 180° the drive pin rides in the level portion of the track to rotate the drive pins and twist the disk positioned therebetween into a smaller overall shape. Subsequently, as the drive member is rotated the additional 180° the drive pin rides in the angled portion of the track thereby drawing the manipulation assembly distally such that the now folded disk is inserted into the sleeve retained on the end of the guide assembly.

Preferably, the track has an extension extending proximally from the longitudinal portion such that advancement of the pin distally in the extension partially ejects the sleeve from the sleeve holder. Preferably, the sleeve holder is provided with a securing screw or knob which frictionally compresses the sleeve holder about the sleeve and upon release of the knob releases the friction on the sleeve.

Manipulation assembly is also provided with a plunger assembly consisting of a plunger proximally biased relative to the drive member by a spring and a cap mounted at the distal end of the plunger. Once the disk has been manipulated to a smaller size and inserted into the sleeve, the plunger can be depressed to move the cap along the pins and against the sleeve to eject the sleeve from the device.

There is also disclosed an insertion tool not forming part of the scope of protection of this patent for use with the now assembled sleeve and manipulated disk. The insertion tool generally includes an outer tube having a throughbore and an outer tube extension extending distally and having a reduced inner diameter which forms a step between the outer tube and the outer tube extension. This step is provided to retain the sleeve within the bore of the outer tube and position the manipulated disk in alignment with the bore of the extension. An inserter is provided to extend through the bore of the outer tube and engage and eject the disk into a prepared disk space.

An alternative embodiment of a manipulation tool not forming part of the scope of protection of this patent is disclosed which is provided to manipulate the disk into a generally elliptical or D-shape for use with an oval cross-section sleeve. The alternate tool includes generally a base and a pair of upwardly extending side supports and a center support movably mounted within the base. A pair of side drive members as well as a vertical drive member are also provided. The disk is generally positioned on top of the side supports and center support and the vertical driver driven to form the disk into an initially C-shaped. Subsequently the side drivers are moved radially inwardly to fold the implant into a generally D-shape.

Methods not forming part of the scope of protection of this patent of using the storage and insertion tool, the storage and manipulation tool, the insertion tool, are also disclosed herein.

There is also disclosed a further alternative embodiment of an implant folding and storage device not forming part of the scope of protection of this patent to fold an implant and store it for use within an insertion device. The disclosed implant folding and storage device generally includes an implant folding device and an implant transfer device and implantation tube configured to be attached to the implant folding device.

The implant folding device generally includes two longitudinally movable jaws mounted on guide members. The jaws define a recess therebetween for receipt of an implant. A drive member is provided to move the jaws relative to each other in order to reduce the size of the recess and compress or fold an implant positioned within the recess.

The implant transfer device is provided to move the folded implant from within the implant folding device and into the implantation tube. The implant transfer device generally includes an outer tube having a locking member at a distal end. The locking member is provided to affix the implant transfer device to the implant folding device. A pusher extends through the tube and is moved by a drive member to force the folded implant out of the implant folding device.

The disclosed implantation tube is provided to be attached to the implant folding device and to store the folded implant. The implantation tube is configured to receive the folded implant by means of the implant transfer device. The implantation tube is attached to the implant folding device by engagement of recesses on the implantation tube with posts on the implant folding device.

A method not forming part of the scope of protection of this patent of using the implant folding and storage device to fold and store an implant is also disclosed. An implant is positioned within the recess between the jaws and the jaws are compressed to fold the implant within the recess. The recess may be configured to fold the implant into a generally C shape or other desired folded configuration. The transfer device is affixed to the folding device and actuated to drive the folded implant into the implantation tube to sleep. Thereafter the implantation tube may be removed and stored for use with insertion instrumentation.

### BRIEF DESCRIPTION OF THE DRAWINGS

Various embodiments are described below with reference to the drawings wherein:
FIG. 1 is a perspective view of a manipulation and storage tool, sleeve and implant;
FIG. 1A is an end view of the manipulation and storage tool;
FIG. 2 a perspective view of the manipulation assembly of the tool of FIG. 1;
FIG. 3A is a perspective view of the manipulation and storage tool with the manipulation assembly rotated 180° relative to a guide assembly;
FIG. 3B is a perspective view of the manipulation and storage tool with the manipulation assembly rotated an additional 180° relative to the guide assembly;
FIG. 3C is a perspective view of the manipulation and storage tool with a plunger depressed to eject a sleeve containing the implant out of the manipulation and storage tool;
FIG. 4A is an end view of an alternative embodiment of a implant manipulation tool;
FIG. 4B is an end view of the tool of FIG. 4A initially folding an implant;
FIG. 4C is an end view of the tool of FIG. 4A further folding the implant;
FIG. 4D is an end view of the folded implant inserted in a sleeve; and
FIG. 5 is a side view, shown in section, of an insertion tool for receiving a sleeve and inserting a folded implant into an intravertebral disk space;
FIG. 6 is a top plan of another embodiment of an implant folding and storage device;
FIG. 7 is a side view of an implant folding device;
FIG. 8 is a side view of an implant transfer device;
FIG. 9 is a side view, partially shown in section, of the implant transfer device;
FIG. 10 is another side view of the implant transfer device;
FIG. 11 is a side view of a drive member of the implant transfer device;
FIG. 12 is a side view of an implantation tube;
FIG. 13 is a perspective view of the implant folding and storage device;
FIG. 14 is a perspective view of the implant folding device with a implant being inserted;
FIG. 15 is a side view of the implant folding device prior to compression of the implant;
FIG. 16 is a top plan review of the impact plant folding device during compression of the implant;
FIG. 17 is a side view of the implant folding device with the implant transfer device attached thereto;
FIG. 18 is a top plan review of the implant being transferred from the implant folding device to the implantation tube;
FIG. 19 is a side view illustrating the folded implant contained in the implantation tube;
FIG. 20 is a perspective view of a modified implant folding device, alternative pusher and transfer tube;
FIG. 21 is a perspective view of the modified implant folding device and transfer tube illustrating alternative connection structure;
FIG. 22 is a perspective view of the transfer tube being attached to the distal end of an implant insertion instrument;
FIG. 23 is a side view, partially shown in section, of the transfer tube prior to affixation to a working sleeve;
FIG. 24 is a perspective view of the working sleeve being connected to the transfer tube;
FIG. 25 is a perspective view of the working sleeve being affixed to the transfer tube;
FIG. 26 is a side elevation view of a surgical instrument or implant insertion tool;
FIG. 27 is an enlarged side view of the handle section of the implant insertion tool partially shown in section; and
FIG. 28 is a side cross-sectional view of another preferred embodiment of the presently disclosed transfer tube.

### DETAELED DESCRIPTION OF PREFERRED EMBODENIENTS

The preferred embodiments of the devices and methods disclosed herein relate to tools for receiving a fully hydrated or fully or partially dehydrated, flexible intervertebral implant and manipulating the implant such that the implant is reduced in overall size and inserted into a storage member or sleeve retained in the tool.

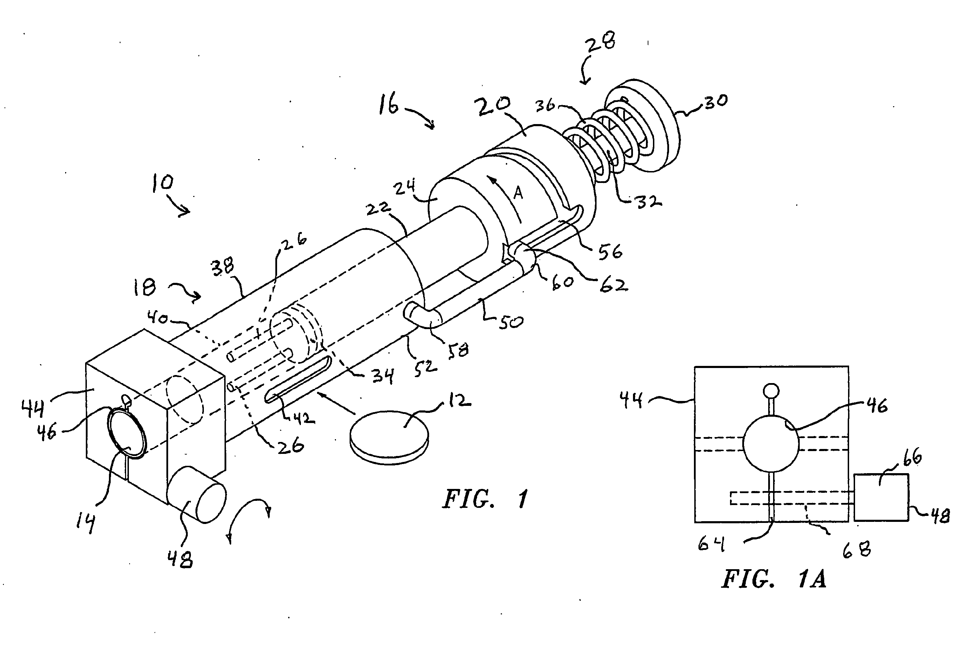

Referring now to FIG. 1, a manipulation and storage tool 10 is provided to receive and manipulate a flexible implant 12 such that it can be inserted into a storage member or sleeve 14 which is retained in manipulation and storage tool 10. Manipulation and storage tool 10 generally includes a manipulation assembly 16 which is configured to manipulate flexible implant 12 into a smaller configuration which may be compressed or folded, etc., such that it may be inserted into sleeve 14. Manipulation and storage tool 10 additionally includes a guide assembly 18 for guiding the flexible implant 12 into sleeve 14.

Manipulation assembly 16 includes a drive member 20 having an outer tube 22 extending distally from distal end 24 of drive member 20. A pair of manipulation members or pins 26 are affixed to a distal end of outer tube 22. Manipulation assembly 16 additionally includes a plunger assembly 28 having a plunge cap 30 and a plunge rod 32 extending distally from plunge cap 30. A pusher 34 is affixed to a distal end of plunge rod 32 to facilitate ejecting sleeve 14 from manipulation and storage tool 10. Preferably plunger assembly 28 is biased in a proximal direction relative to drive member 20 by a spring 36 interposed between plunge cap 30 and drive member 20.

Guide assembly 18 includes a guide tube 38 having a bore 40 therethrough. One or more slots 42 are provided on guide tube 38 and intersect bore 40. Slots 42 are provided to receive flexible implant 12 and position it across bore 40. A sleeve holder 44 is affixed to a distal end of guide tube 38 and includes a bore 46 for receipt retention of sleeve 14. A lock knob 48 is provided to reduce the diameter of bore 46 so as to frictionally engage sleeve 14 and retain it within bore 46. Referring to FIG. 1A, specifically, sleeve holder 44 is sectioned by slot 64 and a threaded rod 68 extends from cap 66 across slot 44a.

Guide assembly 18 and manipulation assembly 16 are interconnected by a drive rod 50. Drive rod 50 extends between a proximal end 52 of guide tube 38 and distal end 24 of drive member 20. Specifically, drive member 20 is provided with a circumferential track 56. A distal end 58 of drive rod 50 is connected to proximal end 52 of guide 38. A proximal end 60 of drive rod 50 is mounted with respect to drive member 20. Specifically, a drive pin 62 formed at the proximal end 60 of drive rod 50 is configured to be retained in and ride within track 56 such that manipulation assembly 16 moves relative to guide assembly 18 in response to rotation of drive member 20.

As noted above, manipulation assembly 16 is provided to manipulate flexible implant 12 into a smaller configuration so that it may be inserted within a storage or sleeve 14. This occurs in response to rotation of manipulation assembly 16, and in particular drive number 20, relative to guide assembly 18. As discussed above, drive pin 62 located at proximal end 60 of drive rod 50 is configured to reside within and move within track 56.

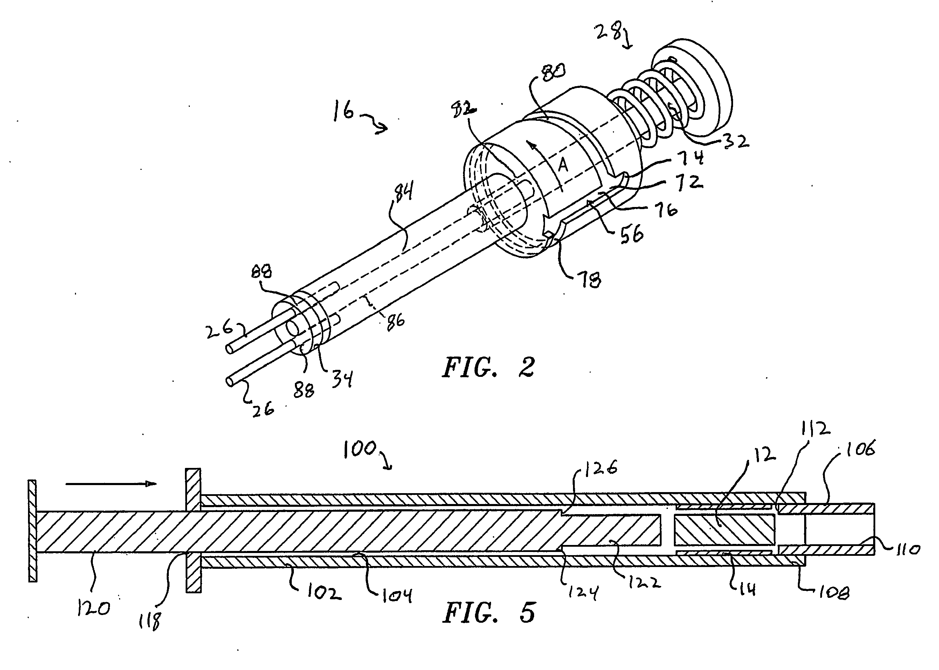

Referring now to FIG. 2, track 56 is provided with a longitudinal portion 76 intersecting a level portion 78 at distal end 54 of drive member 20. Level portion 78 extends circumferentially around drive member 20 approximately 180° where it intersects an angled portion 80 of track 56. Angled portion 80 extends circumferentially 180° from a location adjacent the distal end 54 of drive member 20 proximally to intersect longitudinal portion 76 at a position adjacent proximal end 72 of longitudinal portion 76. An extension 74 of longitudinal portion 76 extends proximally from longitudinal portion 76.

Drive member 20 is provided with a longitudinal bore 82 which connects to a longitudinal bore 84 in outer tube 22. As noted above, plunger assembly 28 includes a plunge rod 32. Plunge rod 32 extends through bores 82 and 84 and drive member 20 and outer tube 22, respectively. Plunger rod 32 is provided with a plunger rod extension 86 which extends through bore 84 and is affixed to pusher 34 at a distal end of plunge rod extension 86. Bores 88 formed in pusher 34 accommodate pins 26 such that upon depression of plunge cap 30, pusher 34 rides distally along pins 26 to engage and expel sleeve 14 from manipulation and storage tool 10.

In using manipulation and storage tool 10 to reduce the overall size of a flexible implant 12 and insert it into a sleeve 14 for storage, tool 10 is initially positioned with manipulation assembly 16 in a proximal most position with respect to guide assembly 18. Plunger assembly 28 is biased to a proximal most position with respect to drive member 20 by spring 36. In this position, drive pin 62 is located at the distal end of longitudinal portion 76 of circumferential track 56. Pins 26 are positioned adjacent slot 42 in a position to receive disk 12 therebetween.

Referring to FIGS. 1 and 1A, a bore 46 of sleeve holder 44 is initially in a relaxed position for sliding receipt of sleeve 14. Sleeve 14 is positioned within bore 46 and lock knob 48 is rotated such that threaded rod 68 is rotated to reduce slot 64 thereby frictionally engaging sleeve 14 within bore 46.

Implant 12 is inserted through 42 such that implant 12 resides within bore 40 and between pins 26.

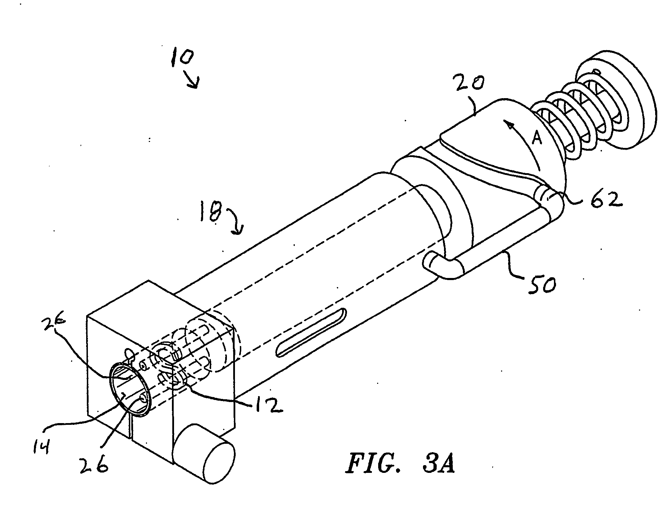

To fold implant 12 into a generally S-shaped configuration drive member 20 is rotated to move drive pin 62 along level portion 78 of circumferential track 56 approximately 180°. This rotates pins 26 causing them to engage disk 12 and form it into a generally S-shape as best seen in FIG. 3A. It should be noted that other shapes such as G-shape or oval, etc. can be provided based on the configuration of the pins 26.

Referring to FIG. 3A, once disk 12 has been manipulated into a generally S-shape, drive member 20 is rotated an additional 180° such that drive pin 62 advances up angled portion 80 of circumferential track 62 an additional 180°. This movement draws manipulation assembly 16 distally relative to guide assembly 18. As manipulation assembly 16 moves distally pins 26 and outer tube 22 carry the now S-shaped disk 12 distally to a position within sleeve 14.

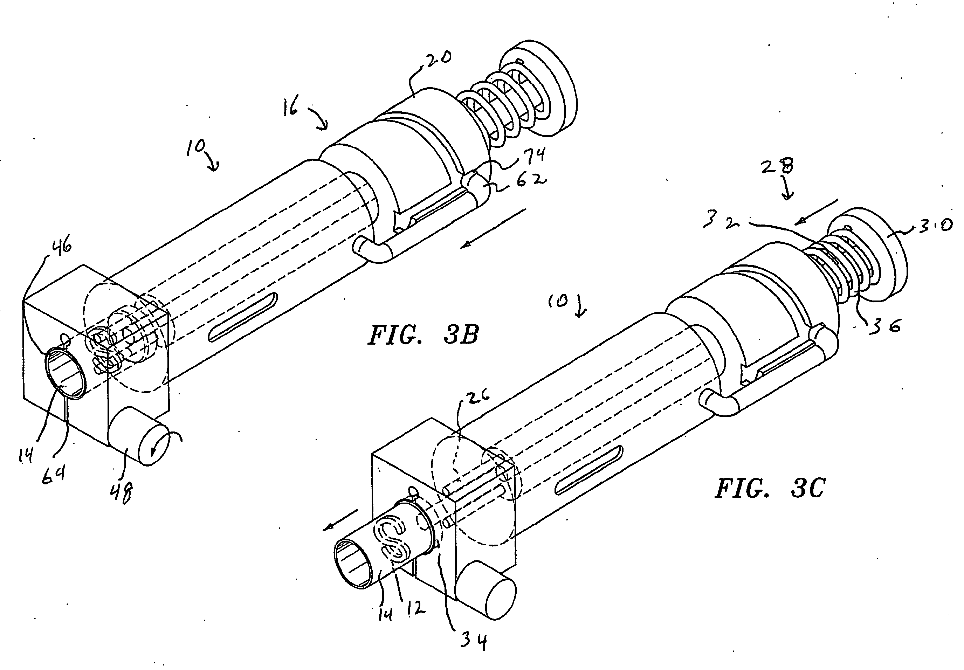

Referring now to FIG. 3B, in order to eject the now assembled sleeve 14 and disk 12 from tool 10, knob 48 is rotated to relax slot 64 such that bore 46 expands and releases sleeve 14. Drive member 20 is advanced an additional distal amount such that drive pin 62 moves into extension 74 thereby moving the entire manipulation assembly 16 distally a slight amount to engage sleeve 14 and move sleeve 14 slightly distally within bore 46 to relieve any frictional engagement between walls of sleeve 14 and bore 46.

Referring now to FIG. 3C, plunger assembly 28 is manipulated to eject sleeve 14 and disk 12 from tool 10. Specifically, plunge cap 30 and plunge rod 32 are moved distally against the bias of spring 36 to drive pusher 34 distally over pins 26. This engages pusher 34 with sleeve 14 and moves sleeve 14 distally. Pusher 34 slides along pins 26 to push implant 12 and sleeve 14 distally. This ensures pusher 34 disengages any frictional contact between implant 12 and pins 26 as sleeve 14 is being ejected from tool 10.

Once flexible disk 12 has been inserted into sleeve 14 by use of manipulation and storage tool 10, the assembled sleeve 14 and disk 12 may be stored for a period of time until needed. As noted, various type, sizes and compositions of implants 12 may be provided to a surgeon so that he or she can choose between various sizes and configurations of implants during an operation.

Referring now to FIG. 5 there is illustrated an insertion tool 100 for use with the now loaded or combined sleeve 14 and gel disk or implant 12. Insertion instrument 100 is configured to the position adjacent the opening in the disk space and utilized to eject the gel disk 12 from sleeve 14 and into the disk space. Specifically insertion tool 100 includes an outer tube 102 defining a throughbore 104. An outer tube extension 106 having an outer diameter slightly less than or equal to the inner diameter of bore 104 extends from a distal end 108 of outer tube 102. Outer tube extension 106 defines a bore 110 which has an inner diameter substantially equal to the inner diameter of sleeve 14. By using the reduced diameter outer tube extension 106 a proximal edge 112 of outer tube extension 106 forms a step or stop against which sleeve 14 can rest and be restrained within bore 104 of outer tube 102.

Insertion tool 100 additionally includes an inserter 120 which is configured to be slidingly received through opening 118 within bore 104 of outer tube 102. Preferably, the diameter of inserter 120 is substantially equal to the diameter of bore 104 to ensure a precise sliding fit with little wobble. Inserter 120 has a reduced diameter portion 122 extending distally from a distal end 124 of inserter 120. The reduced diameter portion forms a step 126. The outer diameter of reduced diameter portion 122 is smaller in diameter than the inner diameter of sleeve 14. However, the outer diameter of inserter 120 is greater in diameter than the inner diameter of outer tube extension 106 such that upon distal advancement of inserter 120 within outer tube 102 step 126 engages proximal edge 112 thereby preventing any further forward motion. The outer diameter of reduced diameter portion 122 is smaller than the inner diameter of sleeve 14 so as to allow reduced diameter portion 122 to push or eject implant 12 through bore110 and into an invertebral disk space.

While not specifically illustrated, the use of insertion tool 100 to receive a loaded sleeve 14 and disk 12 and to insert disk 12 into an intervertebral disk space will now be briefly described. Initially the vertebrae and damaged disk is accessed using known surgical procedures. The annulus of the intervertebral disk is then punctured or excised to expose the nucleus and a portion of the nucleus material is removed, preferably without trauma to the vertebral end plates, resulting in an intervertebral disk space. Various instruments may be utilized to determine the proper restored height for the intervertebral disk spacing. Once the proper height is determined the surgeon can choose between the proper size disk 12 to be inserted into the intervertebral disk space. As noted above, the intervertebral disks and sleeves 14 may be provided to the surgeon in varying heights either preassembled or assembled during the surgery using manipulation and storage tool 10. Once the proper loaded sleeve 14 and disk 12 are obtained, they are assembled in insertion tool 100 by passing loaded sleeve 14 through opening 118 and into bore 104 of outer tube 102. Loaded sleeve 14 slides within bore 104 distally until a distal-most edge of sleeve 14 contacts proximal edge 112 of extension 106. This places disk 12 in alignment with bore 110 of extension 106. Thereafter, inserter 120 is positioned through opening 118 and into bore 104 tool position just proximal of disk 12.

Once insertion tool 100 has been loaded with sleeve 14 and disk 12, insertion tool 100 is positioned such that extension 106 enters the annulus of the disk space and a distal-most edge of distal end 108 is adjacent to and contacts the adjacent vertebra spanning the now excised intervertebral disk space. Once properly positioned to the surgeon's satisfaction, inserter 120 can be advanced distally to cause reduced diameter portion 122 to engage disk 12 and drive disk 12 through sleeve 14 and bore 110 in extension 106 and into the intervertebral disk space. As noted above, step 126 in inserter 120 engages a proximal- most edge 112 of extension 106 to limit the forward advancement of inserter 120 within outer tube 102. Once disk 12 has been properly positioned within the intervertebral disk space, inserter 100 is removed from the disk space and the partially dehydrated disk 12 is allowed to re-hydrate and expand to its proper circular or disk shaped orientation and enlarged to the proper height to maintain the restored disk spacing as desired.

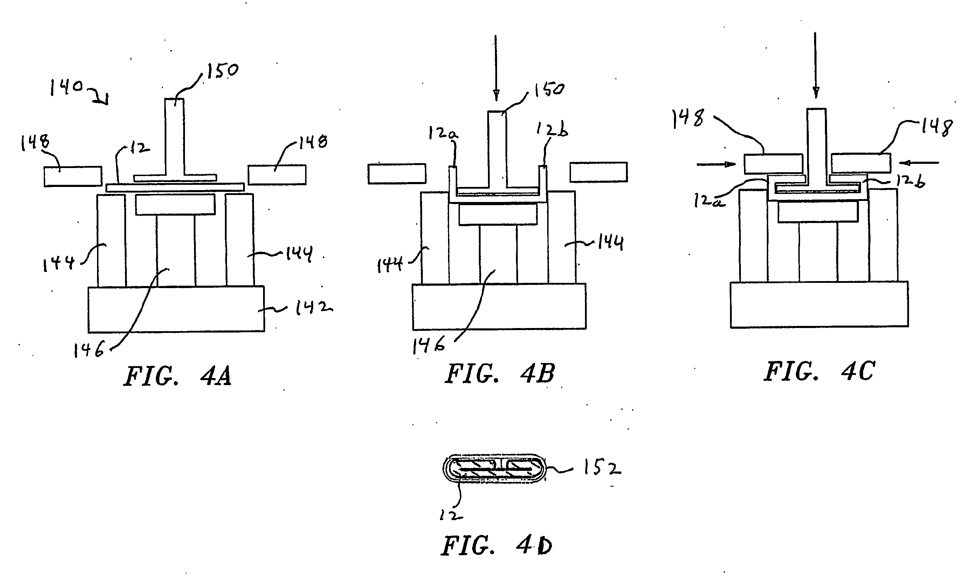

Referring now to FIG. 4A there is disclosed an alternate embodiment of a manipulation tool for forming an implant 12 into a generally folded or elliptical shape. Specifically, manipulation tool 140 generally includes a base 142 having a pair of side supports 144 extending vertically up from base 142. A T-shaped center support 146 is movably mounted with respect to base 142. Horizontal side drivers 148 are provided adjacent side supports 144 to fold outer edges of implant 12. Tool 140 also includes a vertical driver 150 positioned opposite center support 146.

In use, partially dehydrated gel disc or implant 12 is initially positioned on top of center support 146 and side supports 144.

Referring to FIG. 4B, vertical driver 150 is driven downwardly against implant 12 and center support 146 driving center support 146 downwardly such that side support 144, fold outer edges 12a and 12b of implant 12 about vertical driver 150.

Referring to FIG. 4C, once edges 12a and 12b have been folded to in a vertical position side drivers 148 move radially inwardly to fold edges 12a and 12b into a generally block-C or elliptical shape.

Once implant 12 has been so formed by vertical driver 150 moving in an X direction and side drivers 148 moving in a Y direction, an ejector not shown may be advanced in the Z direction to eject folded implant 12 out of manipulation tool 140. Preferably folded implant 12 is ejected or inserted into an over sleeve 152 (FIG. 4D) for use in a similar manner to that of combined sleeve 14 and S-shaped implant 12 hereinabove.

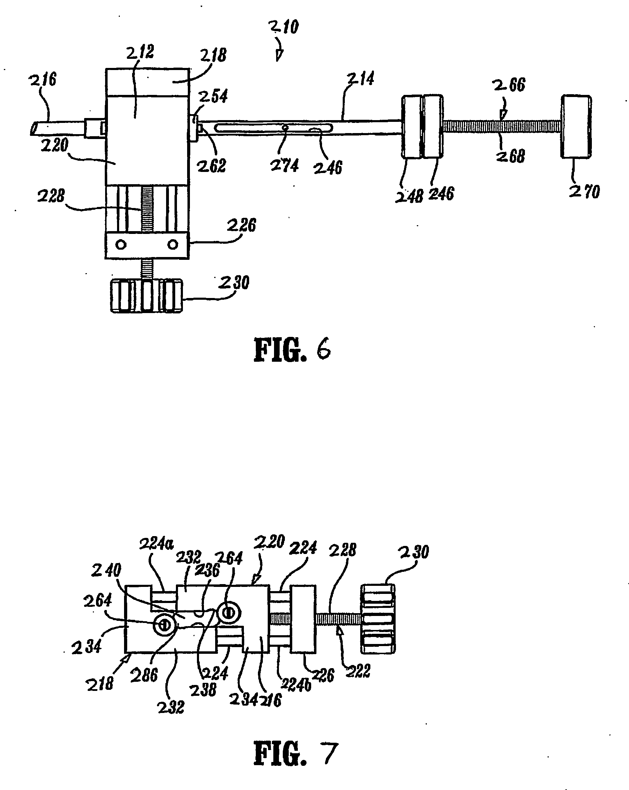

FIGS. 6 and 7 illustrate an alternate embodiment of the presently disclosed implant folding and storage device shown generally as device 210. Device 210 includes an implant folding device 212, an implant transfer device 214, and an implantation storage tube 216.

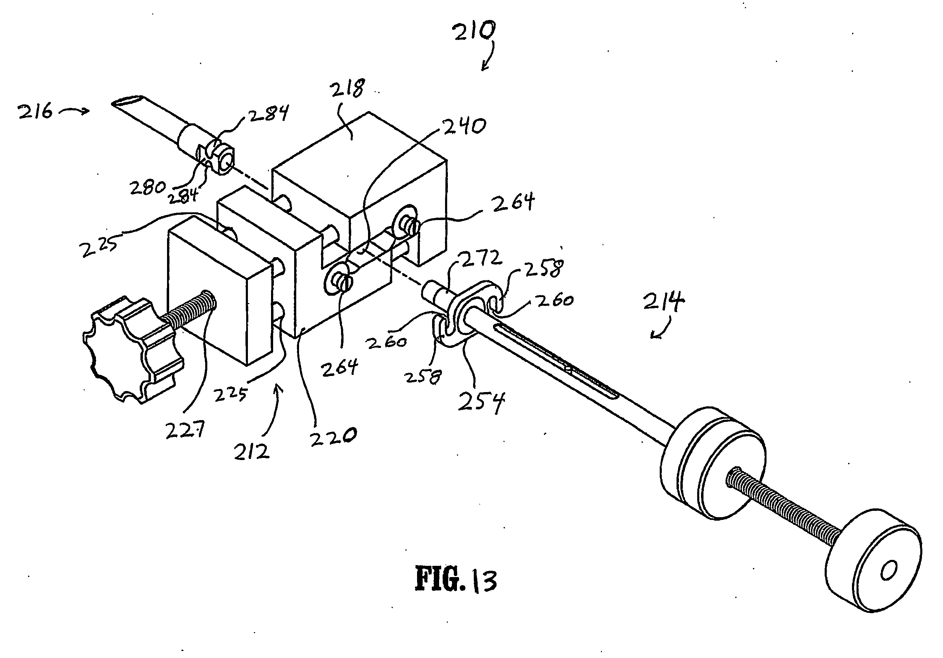

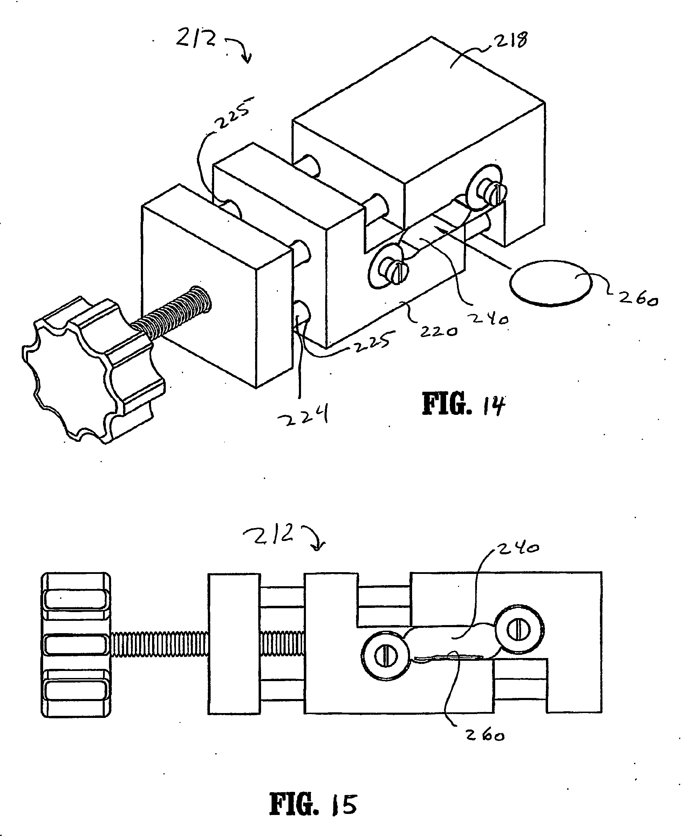

Implant folding device 212 includes a first jaw 218, a second jaw 220 and a drive member 222. First and second jaws 218 and 220 are movably supported in relation to each other on four guide members 224. Alternatively, two guide members may be used. A first end 224a of guide members 224 is axially fixed to first jaw 218. A second end 224b of guide members 224 is axially fixed to a support block member 226. Second jaw 220 includes a plurality of longitudinal throughbores 225 (FIGS. 13 and 14) dimensioned to slidably receive guide members 224. Second jaw 220 is slidably positioned on guide members 224 between first jaw 218 and support block 226. Drive member 222 includes a threaded screw portion 228 and a rotation knob 230 fixedly secured to a proximal end of screw portion 228. Support block 226 includes threaded bore 227 (FIG. 13) dimensioned to rotatably receive screw portion 228 of drive member 222. Preferably, the distal end of screw portion 228 is axially fixed but rotatable with respect to second jaw 220. Alternately, the distal end of screw portion 228 may abut against a sidewall of jaw 220.

In use, when rotation knob 230 is rotated to turn screw portion 228 within the threaded bore in support block 226, screw portion 228 translates axially in relation to support block 226 to move second jaw 220 in relation to first jaw 218 between spaced and approximated positions. Although implant folding device 212 is illustrated as having a drive member in the form of a screw portion, it is envisioned that other known drive members or assemblies may also be used to move the first jaw member in relation to the second jaw member, e.g., ratchet drive mechanisms, levers, pneumatic pistons, etc.

First and second jaws 218 and 220 of implant folding device 212 preferably have a substantially L-shaped configuration including a longitudinally extending leg 232 and a transversely extending leg 234. When jaws 218 and 220 are in an approximated condition, the jaws 218 and 220 interengage to define a substantially rectangular shape. Alternately, the jaws may assume different configurations including circular, square, triangular, etc. Each jaw includes a substantially semi-circular recess 238 formed along an inner wall 236 of longitudinally extending leg 232. Walls 236 of legs 232 and semi-circular recesses 238 together define a receiving chamber 240 for receiving a flexible implant (not shown). When the jaws are fully approximated, recesses 238 define a circular bore. It is envisioned that recesses 238 may be defined by removable plates which can be selectively replaced to change the diameter of the bore defined by recesses 238. Such would allow folding device 210 to be used with different size flexible implants.

In use, when a flexible implant is positioned within receiving chamber 240 and drive member 222 is actuated to approximate jaws 218 and 220, the flexible implant, which may have a normally rectangular or circular configuration, is folded, via engagement with semi-circular recesses 238, into a circular or cylindrical configuration. It is envisioned that the configuration of recesses 238 may be changed to provide different fold patterns for the flexible implant, e.g., S-shape, etc.

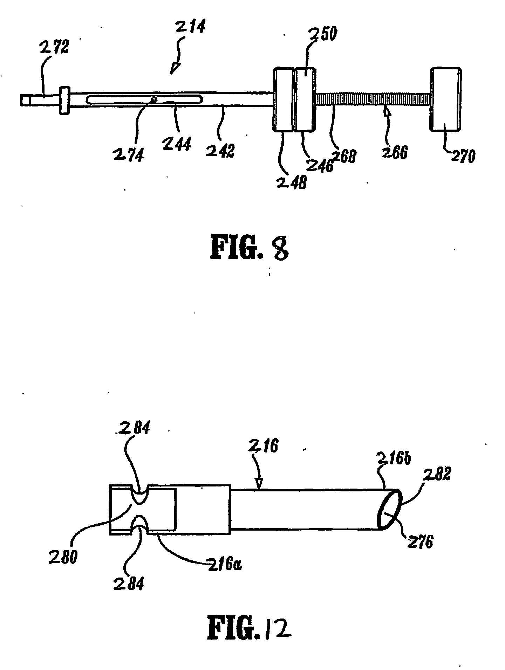

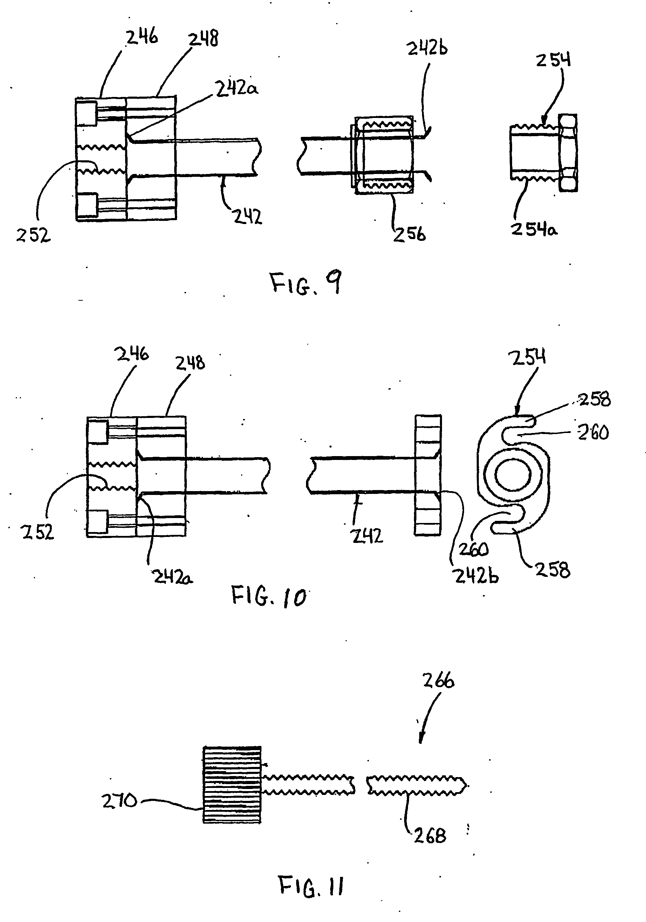

Referring to FIGS. 8-11, transfer device 214 includes an outer tube 242 having an elongated longitudinal slot 244 formed therein. A first end 242a of tube 242 is flared outwardly (FIG. 9). A pair of rings 246 and 248 are secured to first end 242a of tube 242. Rings 246 and 248 each have a knurled outer surface 250 to facilitate gripping of the device. Ring 246 includes an internally threaded bore 252 and is positioned on one side of flared first end 242a of tube 242. Ring 248 is positioned about tube 242 on an opposite side of flared first end 242a. Rings 246 and 248 are secured together, e.g., clamped about flared end 242a of tube 242, to secure the ring assembly to the first end of outer tube 242.

A locking member 254 is secured to a second end of outer tube 242. Locking member 254 includes a proximally threaded extension 254a which is dimensioned to threadably engage the internal threads of a nut 256. The second end of outer tube 242 also includes an outwardly flared portion 242b which is clamped between locking member 254 and nut 256 to secure the locking member to the second end of outer tube 242.

Locking member 254 includes a pair of hook portions 258, each defining a recess 260. Recesses 260 are dimensioned to receive screws 264 supported on implant folding device 212 (FIG. 7) to secure implant transfer device 214 in fixed relation with respect to implant folding device 212. Implant transfer device 214 is secured to implant folding device 212 at a position adjacent a first side of receiving chamber 240. Posts 262 preferably extended through and from each jaw 218 and 220 and are secured thereto by screws 264. Alternately, other securement methods may be used to secure the posts to jaws 218 and 220 or the parts may be monolithically formed with each jaw. Moreover, other known securement methods or devices may be used to secure implant transfer device 214 to implant folding device 212, e.g., bayonet coupling, Luer coupling, screws, etc.

A drive member 266 includes a threaded body 268 and a gripping head 270 secured to body 268. Threaded body 268 is rotatably received within internally threaded bore 252 of ring 246. The distal end of threaded body 268 is positioned in abutting relationship or, alternately, axially fixed to a pusher 272 which is slidably positioned within outer tube 242 of implant transfer device 214. Pusher 272 includes a radially extending pin 274 which is slidably positioned within longitudinal slot 244 of outer tube 242. When drive member 266 is actuated, e.g., rotated in relation to ring 246, pusher 272 is translated within outer tube 244 such that the distal end of pusher 272 extends through a first side of receiving chamber 240 of implant folding device 212 to eject a folded implant from a second side of receiving chamber 240. Radially extending pin 274 provides an external indication of the position of pusher 272 within outer tube 242 and thus, within receiving chamber 240.

Referring to FIG. 12, implantation tube 216 defines an implant storage chamber 276 and includes a proximal end 216a having a pair of flats 280 and a distal end 216b having an angled annular tip 282. A pair of recesses 284 are formed between flats 280 on proximal end 216a of implantation tube 216.

Implant folding device 212 defines an opening 286 (FIG. 7) adjacent a second side of receiving chamber 240. Opening 286 includes flat upper and lower walls 236a and is configured when the jaws 218 and 220 are in the approximated position to slidably receive the proximal end of implantation tube 216. After inserting the proximal end of implantation tube into opening 286, the implantation tube 216 can be rotated to move recesses 284 about posts 262 to lock implantation tube 216 adjacent the second side of receiving chamber 240. Preferably, when the jaws are fully approximated the internal bore of folding device 212 defined between recesses 238 is slightly smaller in diameter than the diameter of storage chamber 276 of implantation tube 216. More preferably, the internal bore of folding device 212 defined between recesses 238 is about .05mm smaller in diameter than the diameter of storage chamber 276. The distal end of pusher 276 is also preferably less than about .25mm in diameter smaller than the internal bore defined between recesses 238 of jaws 218 and 220 when the jaws are in their approximated position.

Referring to Fig. 13, the assembly of the three main components of the implant folding and storage device will now be described. While implant folding and storage device 210 is shown with jaws 220 and 218 in their spaced apart positions, it should be noted that the three components are not assembled until after an implant has been inserted in recess 240 and jaws 218 and 220 approximated to fold the implant. Thereafter, implant transfer device 214 is assembled to implant folding device 212 by initially inserting pusher 272 into recess 240. Implant transfer device 214 is then rotated such that recesses 260 of hook portions 258 engage screws 264 on implant folding device to 212. This securely affixes implant transfer device 214 to implant folding device 212. Thereafter, implantation tube 216 is inserted into recess 240 on an opposite side of implant folding device 212 and rotated such that flats 280 are aligned with posts 262. Thereafter implantation tube 216 is rotated such that recesses 284 engage posts 262 to secure implantation tube 216 to implant folding device 212.

Generally, in use, implant folding device 212, transfer device 214 and implantation tube 216 are assembled in the manner discussed above to define an integral unit. Alternately, either or both of implant transfer device 214 and implantation tube 216 can be secured to implant folding device 212 after the flexible implant has been folded.

Referring now to FIGS. 14 through 19, the operation of implant folding and storage device to fold an implant and insert the implant into implantation tube 216 will now be described. Referring now to FIG. 14, initially, implant folding device 212 is situated such that first and second jaws 218 and 220 are in their spaced apart condition to defining the oval recess 240. The proper size implant 260 is chosen and inserted into recess 240 such that implant 260 lies in a flat condition at shown in FIG. 15.

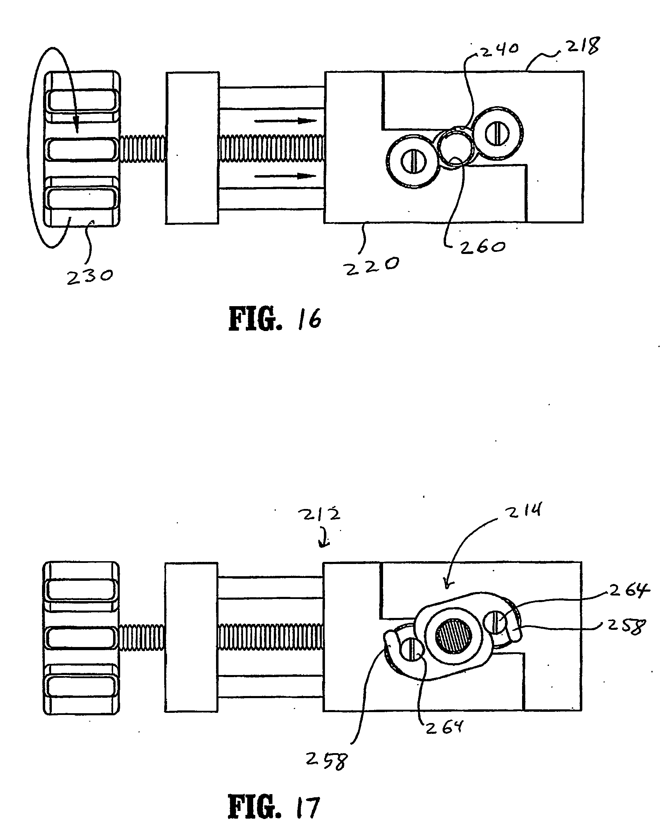

Referring to FIG. 16, rotation knob 230 is rotated to move first and second jaws 218, 220 together thereby shrinking the size of recess 240 and compressing implant 260 into a folded configuration. As noted above, the overall shape of recess 240 may be configured to compress the implant into a generally C shape O shape or other desired shape. As shown in FIG. 17 implant transfer device 214 is affixed to implant folding device 212 by engaging screws 264 with hooks 258. Implantation tube is affixed to an opposite side of implant folding device 212 in the manner described hereinabove.

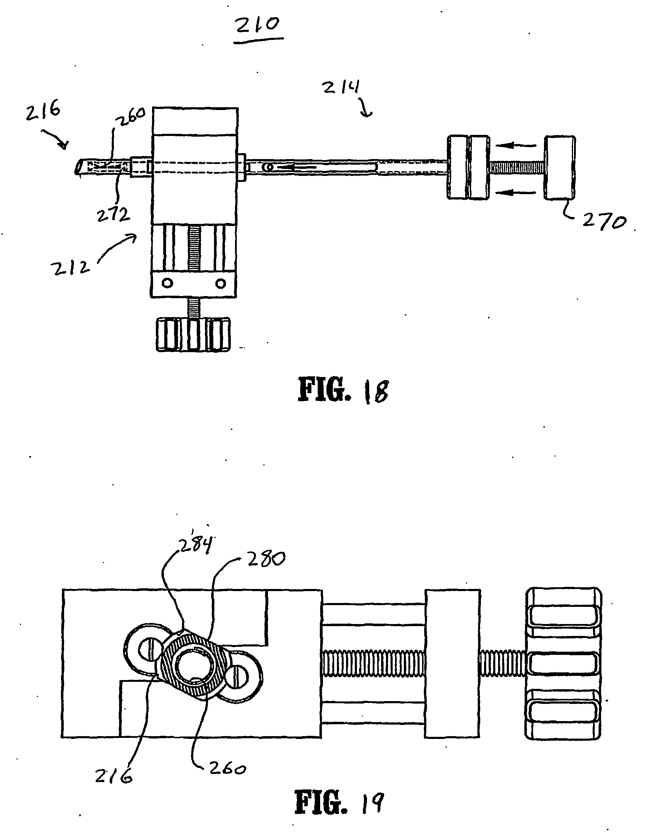

Once implant folding and storage 210 device has been assembled gripping head 270 is rotated to drive pusher 272 through recess 240 thereby forcing folded implant 260 into implantation tube 216 as shown in FIG. 18. Referring to FIG. 19, the folded implant 260 is shown positioned within implantation tube 16.

Once folded implant 260 has been inserted in implantation tube 216, implantation tube 216 may then be removed from implant folding device 212 and immediately used with a surgical instrument or stored for later use.

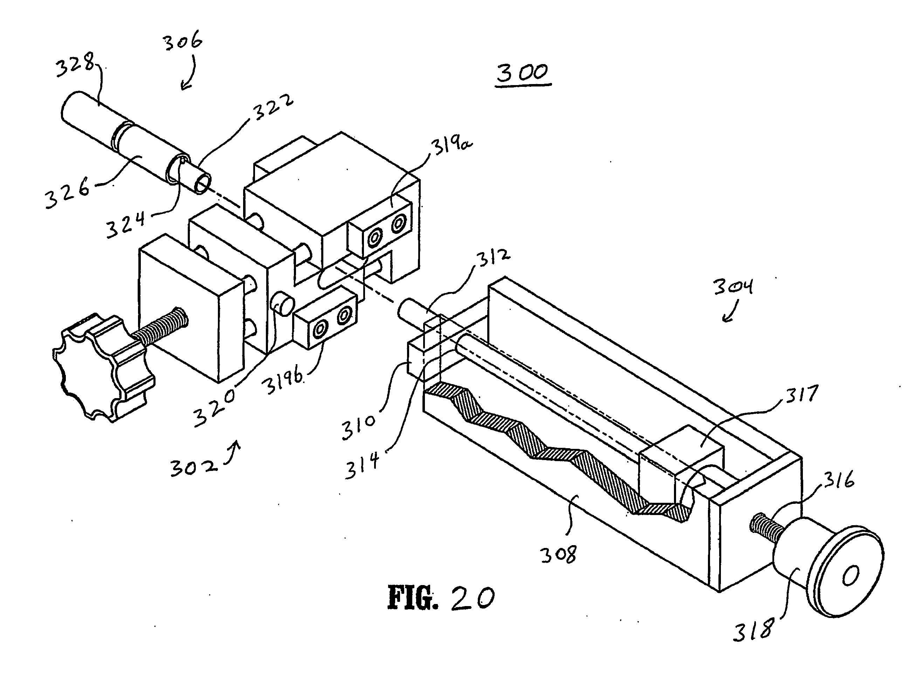

Referring now to FIG. 20, there is disclosed a further alternative embodiment of an implant folding and transfer device 300. Implant folding and transfer device 300 generally includes a modified implant folding device 302, which is substantially similar to implant folding device 212 described in detail hereinabove. Implant folding and transfer device 300 additionally includes an alternative implant pusher 304 which is substantially similar to implant transfer device 214 described hereinabove. Additionally, implant folding and transfer device 300 includes a novel storage or transfer tube 306. Implant folding and transfer device 300 functions in a substantially similar manner to that of implant folding and storage device 210 described hereinabove to fold and store a flexible implant for use with a surgical instrument.

Implant pusher 304 generally includes an elongated frame 308 and a mounting bar 310 positioned at a distal end of elongated frame 308. Mounting bar 310 is configured to engage corresponding structure formed on modified implant folding device 302 to secure implant pusher 304 to implant folding device 302. A pusher 312 is movably mounted within elongated frame 308 and extends through a bar hole 314 in mounting bar 310. Pusher 312 functions in a substantially similar manner to that of the pusher described hereinabove to move a folded implant out of a recess in the folding device and into a transfer or storage tube. A threaded drive rod 316 extends through proximal and of elongated frame 308 and is rotatably mounted through a mounting block 317 and connected at its distal end to pusher 312. A knob 318 is affixed to a distal of threaded drive ride 316. Rotation of knob 318 moves pusher 312 through the recess to eject a folded implant from the recess in the folding device.

As noted hereinabove, implant folding device 302 includes modified structure to receive and affix implant pusher 304. The implant folding device 302 includes blocks 319 and 319b mounted adjacent the recess to slidably received mounting bar 310 of implant pusher 304. A detent 320 is provided on implant folding device 302 to lock implant pusher 304 to implant folding device 302.

Novel transfer tube 306 is provided to receive and store a folded implant from implant folding device 302 and to be attached to a novel implant insertion tool for insertion of the folded implant into the body. Transfer tube 306 includes structure to engage corresponding structure on implant folding device 302 to secure transfer tube 306 to implant folding device 302. Transfer tube 306 includes transfer sleeve 322 which is designed to be received in the recess of implant folding device 302. A guide pin 324 is provided on transfer sleeve 322 and a rotary first nut 326 is provided to secure transfer tube 306 to implant folding device 302. A second nut 328 is also provided to affix transfer tube 306 to a working sleeve.

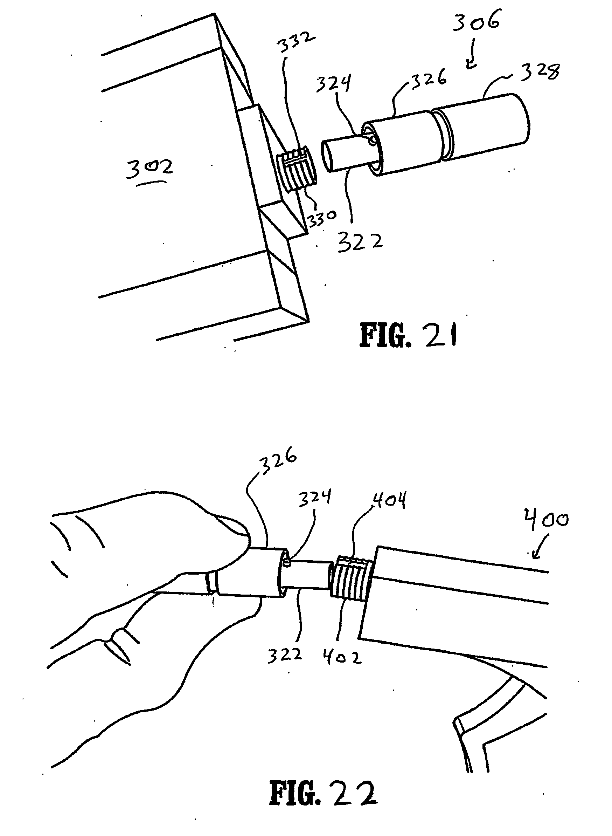

Referring now to FIGS. 21 and 22, the connections of transfer tube 306 to implant folding device 302 and novel implant insertion tool 400 will now be described. Initially, with regard to FIG. 21, transfer tube 306 is positioned adjacent implant folding device 300 such that transfer sleeve 322 and guide pin 324 are adjacent implant folding device 302. Transfer sleeve 322 is inserted within a threaded tube 330 formed on implant folding device 302. Guide pin 324 is aligned with and slid into a guide slot 332 formed on threaded tube 330. Thereafter first nut 326 is rotated such that the threads in the first nut 326 engage threaded tube 330 to secure transfer tube 306 to implant folding device 302.

Referring now to FIG. 22, a similar manner of attachment is used to attach transfer tube 306 to the novel implant insertion instrument 400, after a folded implant has been inserted into transfer tube 306, and transfer tube 306 has been removed from implant folding device 302. Specifically, transfer sleeve 322 is slid into a threaded tube 402 formed on a distal end of insertion instrument 400 and guide pin 324 is aligned with a slot 404 formed in threaded tube 402. Thereafter, first nut 326 is rotated such that the threads in first nut 326 engage the threads of threaded tube 402 to securely affix transfer tube 306 to insertion tool 400.

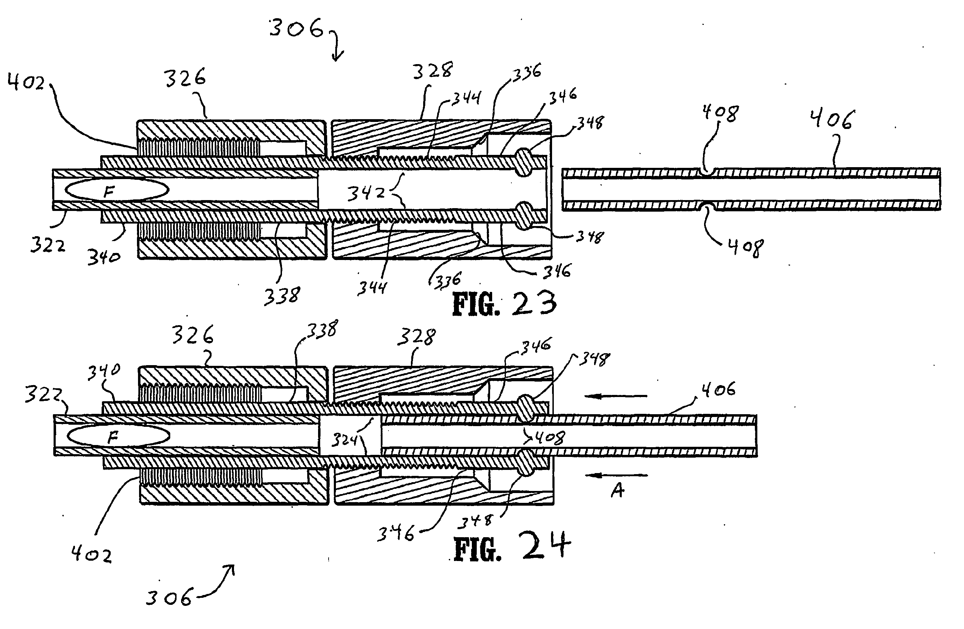

Referring now FIGS. 23 to 25, the novel transfer tube 306 and its attachment to implant insertion tool 400 will now be described. Second nut 328 includes an enlarged bore 334 having camming surfaces 336 formed therein. A connector tube 338 is positioned within first and second nuts 326 and 328, respectively, and is positioned about transfer sleeve 322. Connector tube 338 generally includes a proximal section 340 positioned between first nut 326 and transfer sleeve 322. A plurality of flexible beams 342 extend distally from proximal section 340. Preferably, connector tube 338 includes at least three flexible beams 342. Flexible beams 342 include proximal threaded portions 344 and distal lock portions 346. Lock portions 346 include bumps 348 which are configured to engage corresponding structure on the distal end of a surgical implant instrument Bumps 348 engage camming surfaces 336 on second nut 328 to force lock portions 346 against the distal portion of the surgical instrument.

The novel surgical instrument disclosed therein is configured to be used with a hollow working sleeve 406 which is provided to guide and insert the folded implant into the body. The working sleeve 406 is provided with slots or concavities 408 to receive bumps 348 on transfer tube 306.

Referring now to FIG. 24, in order to affix working sleeve 406 to transfer tube 306, working sleeve 406 is positioned adjacent transfer tube 306 and is slid into connector tube 338 in the direction of arrow A as shown. Working sleeve 406 is positioned within transfer tube 306 until bumps 348 on lock portions 346 engage slots 408.

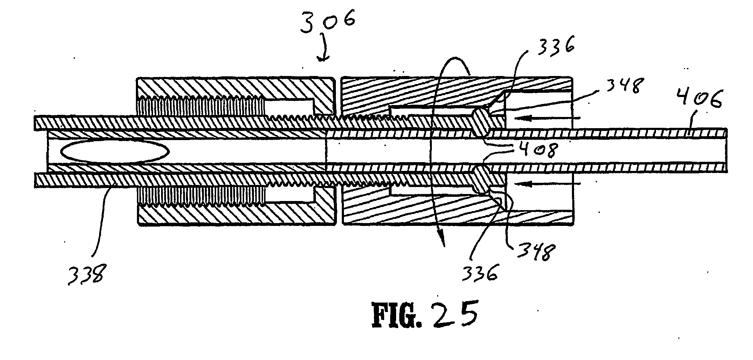

In order to securely lock working sleeve 406 within transfer tube 306 it is necessary to secure bumps 348 within slots 408. Referring FIG. 25, second nut 328 is rotated along threaded portion 344 of connector tube 338 until camming surfaces 336 engage bumps 348. This securely lock bumps 348 within slots 408 and prevents any accidental release of working sleeve 406 from transfer tube 306.

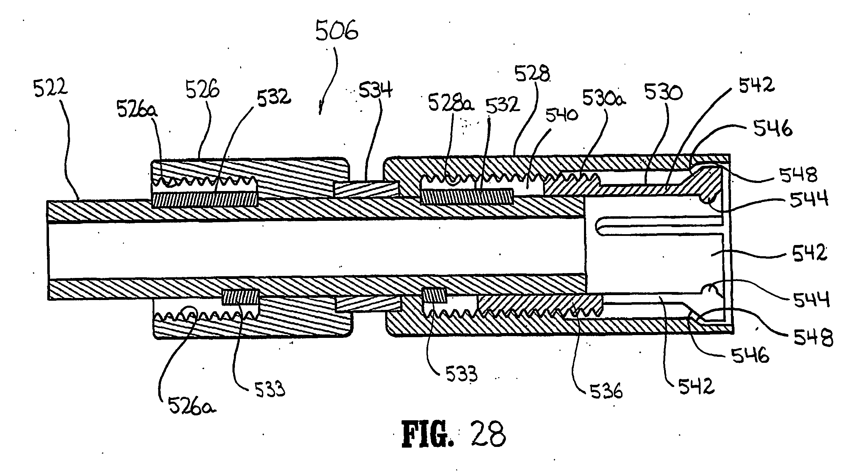

FIG. 28 illustrates another preferred embodiment of the presently disclosed transfer tube shown generally as 506. Transfer tube 506 is also provided to receive and store a folded implant from implant folding device 302 and is adapted to be attached to an implant insertion tool in a manner similar to that disclosed above with respect to transfer tube 306. Transfer tube 506 includes a transfer sleeve 522, a first nut 526 rotatably mounted about transfer sleeve 522, a second nut 528 rotatably mounted about an opposite end of transfer sleeve 522, and a clamp ring 530. First and second nuts 526 and 528 are axially fixed on transfer sleeve 522 by locking members 533 which will be described in further detail below. A spacer 534 is positioned between first and second nuts 526 and 528 to maintain proper spacing.

First nut 526 includes a series of internal threads 526a and second nut 528 includes a series of internal threads 528a. Clamp ring 530 includes a cylindrical portion 536 having a series of external threads 530a. Threads 530a are engageable with internal threads 528a of second nut 528 such that when second nut 528 is rotated about transfer sleeve 522, clamp ring 530 is retracted into or extended from second nut 528. Key member 532 is slidably received within a key slot 540 formed in cylindrical portion 536 of clamp ring 530 to properly align clamp ring 530 within second nut 528. A second key member 532a is provided to be received in a key slot (not shown) in the tube 402 of instrument 400.

Clamp ring 530 includes a plurality of flexible beams 542 which extend outwardly from cylindrical portion 536 of clamp ring 530. A convexity or bump 544 is formed on an inner surface of each beam 542. As discussed above with respect to transfer tube 306, convexities 544 are dimensioned to be received within concavities 408 formed in working sleeve 406 to secure working sleeve 406 within clamp ring 530. A raised cam surface 546 is formed on an outer surface of each of beams 542. Cam surfaces 546 are positioned to engage an inner cam surface 548 on second nut 528 to urge beams 542 inwardly such that convexities 544 are pressed into concavities 408 (FIG. 22) of working sleeve 406 to secure working sleeve 406 to transfer tube 506.

In use, working sleeve 406 is inserted into clamp ring 530 such that convexities or bumps 544 are received within concavities 408 of working sleeve 406. Next, second nut 528 is rotated to withdraw clamp ring 530 into second nut 528. As clamp ring 530 moves linearly into second nut 528, cam surface 546 on beams 542 engage cam surface 548 on second nut 528 to urge beams 542 inwardly to lock working sleeve 406 within clamp ring 530. Next, transfer tube 522 is inserted into threaded tube 402 (FIG. 22) formed on a distal end of instrument 400 and first nut 526 is rotated to secure tube 402 to internal threads 526a of first nut 526.

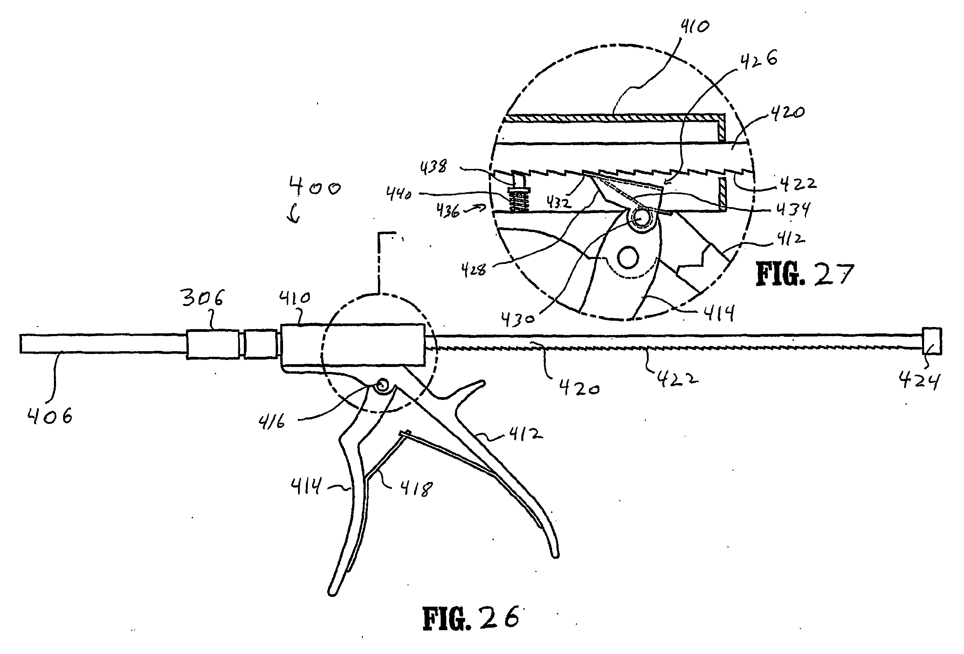

Referring now FIG. 26, there is disclosed a novel surgical instrument assembly for insertion of a folded implant into an area of the body such as, for example, an area of the spinal column. The surgical instrument assembly generally includes surgical instrument 400 along with transfer tube 306 and working sleeve 406. Surgical instrument 400 includes a body portion 410 having a fixed handle 412 extending therefrom. A movable handle 414 is movably mounted to body portion 410 at a pivot at 416. Movable handle 414 is biased away from fixed handle 412 by a leaf spring 418. Surgical instrument 400 also includes a pusher ride 420 which extends through body portion 400 and is configured to advance a flexible implant contained within transfer tube 306 through working sleeve 406 and into the body. By repeatedly moving mobile handle 414 relative to body portion 400 and fixed handle 412, pusher rod 420 is driven through body portion 410 in a manner described below. Pusher rod 420 includes a plurality of ratchet teeth 422 along the length thereof. The pusher rod 420 is also provided with a proximal stop 424 formed at the proximal and of pusher rod 420. Proximal stop 424 limits the advancement of pusher rod 420 through body portion 410 to prevent inadvertent insertion of the pusher rod 420 into the body.

Referring to FIG. 27, a mechanism is provided to prevent the retraction of pusher rod 420. A ratchet mechanism 426 is provided to the rod 420 through housing 410. Ratchet mechanism 426 generally includes a latch 428 pivotally mounted to movable handle 414 by a pivot 430. A forward edge 432 of latch 428 is configured to engage ratchet teeth 422 of pusher rod 420 and move pusher 420 in response to actuation of movable handle 414. Latch 428 is biased against pusher rod 420 by means of a coil spring 434 positioned about pivot 430. Thus, as movable handle 414 is moved proximally against the bias of spring 418 pusher rod 420 is driven distally. Upon release of movable handle 414 latch 428 moves proximally such that forward edge 432 is drawn proximally along teeth 422.

In order to prevent retraction of rod 420 upon release of movable handle 414, there is provided a secondary ratchet 436 which is also engageable with teeth 422 of pusher rod 420. Specifically, secondary ratchet 436 includes a lot number 438 engageable with teeth 422 and a bias spring 440 to bias lock member 438 into engagement with teeth 422. It should be noted that, there may be provided various structure to release ratchet mechanism 426 and secondary ratchet 436 in order to draw pusher rod 420 proximally to reuse surgical instrument 400.

In use, a flexible implant is folded in one of the above described folding apparatus and inserted into transfer tube 306. Transfer tube 336 is assembled to surgical instrument 400 and a manner described hereinabove and working sleeve 406 is assembled to transfer tube 306. The desired area of the patients body is accessed in a known manner and the distal and all of working sleeve 406 is inserted into the patient. Movable handle 414 of surgical instrument 400 is actuated to drive pusher rod 420 and thus the folded implant in transfer sleeve 306 through working sleeve 406 and into the desired area of the body. Surgical instrument 400, along with transfer tube 306 and working sleeve 406, form a novel surgical instrument assembly for insertion of a folded implant into the desired area of the body.

It will be understood that various modifications may be made to the embodiments disclosed herein. With regard to manipulation and storage tool 10 for example, the manipulated shapes of implant 12 and the corresponding shapes sleeve 14 may be altered to facilitate use with different size and shapes of implants 12. Further the drive track could be a continuous helix around the drive member such that the folding of the implant and its insertion into a sleeve occur simultaneously.

Additionally, the disclosed manipulation and storage tool may find use with flexible implants other than intervertebral implants, such as those used in knee surgery, etc. It should also be understood that the disclosed manipulation and storage tool may be fabricated from any material suitable for use in surgery which has the required hardness and durability. Metals and/or polymeric materials are known to those skilled in the art that are frequently used in manufacturing such tools. With regard to implant folding and storage device 210 for example, the particular structure used to secure the components of the device together, e.g., screws, rotatable couplings, etc., may be selected from any known components or techniques without departing from the scope of the invention. Further, the presently disclosed device may be used with a variety of differently shaped and constructed flexible implants. Moreover the implants may be formed from a variety of different types of materials including partially hydrated anisotropic implants.

## Claims

1. A surgical instrument assembly for insertion of a folded intervertebral implant into a body comprising:
a transfer tube (306, 506) for receipt and storage of a folded intervertebral implant (F);
a working sleeve (406) engageable with the distal end of the transfer tube; and
a surgical instrument (400) engageable with the proximal end of the transfer tube and configured to drive the folded intervertebral implant (F) out of the transfer tube, through the working sleeve to and into the body.

2. A surgical instrument assembly according to claim 1 wherein said folded intervertebral implant (F) is S-shaped.

3. A surgical instrument assembly according to claim 1 wherein said folded intervertebral implant (F) is C-shaped.

4. A surgical instrument assembly according to claim 1 wherein said folded intervertebral implant (F) is cylindrically shaped.

5. A surgical instrument assembly according to claim 1 wherein said surgical instrument (400) is configured to drive the folded intervertebral implant (F) out of the transfer tube (306, 506), through the working sleeve (406) to and into the body and into a disc space between vertebrae.

6. A surgical instrument assembly according to claim 1 wherein the transfer tube (306, 506) comprises:
a transfer sleeve (322, 522) having first and second nuts (326, 526), (328, 528) mounted thereon and a lock member for engagement with a working sleeve (406).

7. A surgical instrument assembly according to claim 6 wherein the first nut (326, 526) is threaded for engagement with a folding apparatus (302).

8. A surgical instrument assembly according to claim 6 wherein the transfer sleeve (306) has a guide pin (324) for engagement with the folding apparatus (302).

9. A surgical instrument assembly according to claim 6 wherein the transfer tube (306) further comprises a connector (338) positioned within the first and second nuts (326, 328) and configured to engage a working sleeve (406).

10. A surgical instrument assembly according to claim 6 wherein the transfer tube (306, 506) includes at least one flexible beam (342, 542) configured to be moved against the working sleeve (406).

11. A surgical instrument assembly according to claim 10 wherein the flexible beam (342, 542) includes a bump (348, 548) configured to engage a corresponding structure (408) on the working sleeve (406).

12. A surgical instrument assembly according to claim 10 wherein the second nut (328, 528) includes a camming surface (336, 548) to force the flexible beam (342, 542) against a working sleeve (406).

13. A surgical instrument assembly according to claim 1 wherein the surgical instrument (400) comprises:
a body portion (410) having a fixed handle (412) and a movable handle (414) movably mounted to the body portion; and
a pusher ride (420) movable through the body portion in a response to actuation of the movable handle.

14. A surgical instrument assembly according to claim 13 wherein the pusher ride (420) includes ratchet teeth (422) and the a movable handle (414) includes a latch (428) engageable with the ratchet teeth:

15. A surgical instrument assembly according to claim 13 wherein the surgical instrument (400) further comprises a bias spring (434) to bias the latch (428) into engagement with the ratchet teeth (422).

16. A surgical instrument assembly according to claim 13 wherein the surgical instrument (400) further comprises a secondary ratchet (436) engageable with the teeth (422) to prevent inadvertent retraction of the pusher ride (420).

## Patentansprüche

1. Chirurgische Instrumentenanordnung zum Einsetzen eines zusammengelegten intervertebralen Implantats in einen Körper, welche umfasst:
- ein Transferrohr (306, 506) zur Aufnahme und Lagerung eines zusammengelegten intervertebralen Implantats (F);
- eine Arbeitshülse (406), die mit dem distalen Ende des Transferrohrs einrückbar ist; und
- ein chirurgisches Instrument (400), das mit dem proximalen Ende des Transferrohrs einrückbar ist und zum Treiben des zusammengelegten intervertebralen Implantats (F) aus dem Transferrohr heraus durch die Arbeitshülse zu dem und in den Körper ausgelegt ist.

2. Chirurgische Instrumentenanordnung nach Anspruch 1, **dadurch gekennzeichnet, dass** das zusammengelegte intervertebrale Implantat (F) S-förmig ist.

3. Chirurgische Instrumentenanordnung nach Anspruch 1, **dadurch gekennzeichnet, dass** das zusammengelegte intervertebrale Implantat (F) C-förmig ist.

4. Chirurgische Instrumentenanordnung nach Anspruch 1, **dadurch gekennzeichnet, dass** das zusammengelegte intervertebrale Implantat (F) zylinderförmig ist.

5. Chirurgische Instrumentenanordnung nach Anspruch 1, **dadurch gekennzeichnet, dass** das chirurgische Instrument (400) dafür ausgelegt ist, das zusammengelegte intervertebrale Implantat (F) aus dem Transferrohr (306, 506) durch die Arbeitshülse (406) zu dem und in den Körper und in einen Bandscheibenraum zwischen Wirbeln zu treiben.

6. Chirurgische Instrumentenanordnung nach Anspruch 1, **dadurch gekennzeichnet, dass** das Transferrohr (306, 506) umfasst:
- eine Transferhülse (322, 522) mit daran angebrachten ersten und zweiten Schraubenmuttern (326, 526), (328, 528) und einem Arretierungselement zum Eingriff mit einer Arbeitshülse (406).

7. Chirurgische Instrumentenanordnung nach Anspruch 6, **dadurch gekennzeichnet, dass** die erste Schraubenmutter (326, 526) zum Eingrifft mit einer Zusammenlegvorrichtung (302) mit Gewinde versehen ist.

8. Chirurgische Instrumentenanordnung nach Anspruch 6, **dadurch gekennzeichnet, dass** das Transferrohr (306) einen Führungszapfen (324) zum Eingriff mit der Faltvorrichtung (302) aufweist.

9. Chirurgische Instrumentenanordnung nach Anspruch 6, **dadurch gekennzeichnet, dass** das Transferrohr (306) weiterhin einen in der ersten und zweiten Schraubenmutter (326, 328) positionierten und zum Eingriff mit einer Arbeitshülse (406) ausgelegten Konnektor (338) umfasst.

10. Chirurgische Instrumentenanordnung nach Anspruch 6, **dadurch gekennzeichnet, dass** das Transferrohr (306, 506) mindestens einen biegsamen Träger (342, 542) enthält, der zum Bewegen gegen die Arbeitshülse (406) ausgelegt ist.

11. Chirurgische Instrumentenanordnung nach Anspruch 10, **dadurch gekennzeichnet, dass** der biegsame Träger (342, 542) einen dafür ausgelegten Höcker (348, 548) aufweist, mit einer entsprechenden Struktur (408) an der Arbeitshülse (406) zu greifen.

12. Chirurgische Instrumentenanordnung nach Anspruch 10, **dadurch gekennzeichnet, dass** die zweite Schraubenmutter (328, 528) eine Nockenfläche (336, 548) enthält, um den biegsamen Träger (342, 542) gegen eine Arbeitshülse (406) zu drücken.

13. Chirurgische Instrumentenanordnung nach Anspruch 1, **dadurch gekennzeichnet, dass** das chirurgische Instrument (400) umfasst:
einen Körperteil (410) mit einem festen Griff (412) und einem beweglichen Griff (414), der an dem Körperteil beweglich angebracht ist; und
einen Schieber (420), der als Reaktion auf Betätigung des beweglichen Griffs durch den Körperteil beweglich ist.

14. Chirurgische Instrumentenanordnung nach Anspruch 13, **dadurch gekennzeichnet, dass** der Schieber (420) Ratschenzähne (422) umfasst und der bewegliche Griff (414) eine mit den Ratschenzähnen einrückbare Sperrklinke (428) umfasst.

15. Chirurgische Instrumentenanordnung nach Anspruch 13, **dadurch gekennzeichnet, dass** das chirurgische Instrument (400) weiterhin eine Vorspannfeder (434) zum Vorspannen der Sperrklinke (428) in Eingriff mit den Ratschenzähnen (422) umfasst.

16. Chirurgische Instrumentenanordnung nach Anspruch 13, **dadurch gekennzeichnet, dass** das chirurgische Instrument (400) weiterhin eine sekundäre Ratsche (436) umfasst, die mit den Zähnen (422) einrückbar ist, um ein unabsichtliches Zurückziehen des Schiebers (420) zu verhindern.

## Revendications

1. Ensemble formant instrument chirurgical pour l'insertion d'un implant intervertébral plié dans un corps, comprenant:
un tube de transfert (306,506) pour la réception et le stockage d'un implant intervertébral plié (F);
un manchon de travail (406) pouvant être mis en prise avec l'extrémité distale du tube de transfert; et
un instrument chirurgical (400) pouvant être mis en prise avec l'extrémité proximale du tube de transfert et configuré pour entraîner l'implant intervertébral plié (F) hors du tube de transfert, à travers le manchon de travail et dans le corps.

2. Ensemble formant instrument chirurgical selon la revendication 1, où ledit implant intervertébral plié (F) est configuré en S.

3. Ensemble formant instrument chirurgical selon la revendication 1, où ledit implant intervertébral plié (F) est configuré en C.

4. Ensemble formant instrument chirurgical selon la revendication 1, où ledit implant intervertébral plié (F) a une forme cylindrique.

5. Ensemble formant instrument chirurgical selon la revendication 1, où ledit instrument chirurgical (400) est configuré pour entraîner l'implant intervertébral plié (F) hors du tube de transfert (306,506), à travers le manchon de travail (406) vers et dans le corps et dans un espace de disque entre les vertèbres.

6. Ensemble formant instrument chirurgical selon la revendication 1, où le tube de transfert (306,506) comprend:
un manchon de transfert (322,522) ayant des premier et second écrous (326,526), (328,528) montés sur celui-ci et un élément de verrouillage pour une mise en prise avec un manchon de travail (406).

7. Ensemble formant instrument chirurgical selon la revendication 6, où le premier écrou (326,526) est fileté pour une mise en prise avec un appareil de pliage (302).

8. Ensemble formant instrument chirurgical selon la revendication 6, où le tube de transfert (306) présente un axe de guidage (324) pour une mise en prise avec l'appareil de pliage (302).

9. Ensemble formant instrument chirurgical selon la revendication 6, où le tube de transfert (306) comprend en outre un connecteur (338) positionné dans les premier et second écrous (326,328) et configuré pour une mise en prise avec un manchon de travail (406).

10. Ensemble formant instrument chirurgical selon la revendication 6, où le tube de transfert (306, 506) comprend au moins une poutrelle flexible (342, 542) configurée pour être déplacée contre le manchon de travail (406).

11. Ensemble formant instrument chirurgical selon la revendication 10, où la poutrelle flexible (342, 542) comprend une bosse (348, 548) configurée pour venir en prise avec une structure correspondante (408) sur le manchon de travail (406).

12. Ensemble formant instrument chirurgical selon la revendication 10, où le second écrou (328, 528) comprend une surface de came (336, 548) pour forcer la poutrelle flexible (342, 542) contre un manchon de travail (406).

13. Ensemble formant instrument chirurgical selon la revendication 1, où l'instrument chirurgical (400) comprend:
une portion de corps (410) comportant une poignée fixe (412) et une poignée mobile (414) installée d'une manière mobile sur la portion de corps; et
une course de poussoir (420) déplaçable à travers la portion de corps en réponse à l'actionnement de la poignée mobile.

14. Ensemble formant instrument chirurgical selon la revendication 13, où la course de poussoir (420) comprend des dents d'encliquetage (422), et la poignée mobile (414) comprend un loquet (428) pouvant être mis en prise avec les dents d'encliquetage.

15. Ensemble formant instrument chirurgical selon la revendication 13, où l'instrument chirurgical (400) comprend en outre un ressort de sollicitation (434) pour solliciter le loquet (428) en prise avec les dents d'encliquetage (422).

16. Ensemble formant instrument chirurgical selon la revendication 13, où l'instrument chirurgical (400) comprend en outre un cliquet secondaire (436) pouvant être mis en prise avec les dents (422) pour empêcher un retrait par inadvertance de la course de poussoir (420).
